# EUROPEAN PATENT APPLICATION

(11) **EP 4 703 470 A1**
(43) Date of publication of application: **04.03.2026**
(21) Application number: 24196587.0
(22) Date of filing: 27.08.2024
(51) Int. Cl.: C12N 9/42, C11D 3/386, D06M 16/00

(54) **CELLULASE VARIANTS**

(71) Applicant: AB Enzymes Finland Oy, 05200 Rajamäki (FI)
(72) Inventor: JUNTUNEN, Kari, 05200 Rajamäki (FI); ALAPURANEN, Marika, 05200 Rajamäki (FI); LEHTINEN, Satu, 05200 Rajamäki (FI); MÄKINEN, Susanna, 05200 Rajamäki (FI); OJA, Merja, 05200 Rajamäki (FI); HELLMUTH, Hendrik, 64293 Darmstadt (DE); PURANEN, Terhi, 05200 Rajamäki (FI)
(74) Representative: Espatent Oy

(57) **Abstract**

A cellulase variant comprising at least one structural modification selected from a substitution in the active site and truncation of a loop is provided, and a detergent comprising the cellulase variant and a method of producing the cellulase variant and a method of using the cellulase variant to treat fabric.

## Description

### FIELD OF THE INVENTION

The present invention relates to cellulase variants, to products containing the cellulase variants, and methods for manufacturing them.

### BACKGROUND OF THE INVENTION

Cellulases or cellulolytic enzymes are enzymes involved in hydrolysis of cellulose. In the textile industry, cellulases are used in denim finishing to create a fashionable stone washed appearance in denim cloths in a biostoning process, and they are also used to clean fuzz and prevent formation of pills on the surface of cotton garments. In detergent industry cellulases are used to brighten colors and to prevent graying, pilling of garments, and to improve cleaning. Cellulases are further used in food industry and animal feed manufacturing, and they have a great potential in biomass hydrolysis and in the pulp and paper industry, where they can increase fibrillation, or support deinking, by being added e.g. after cooking and before bleaching in a Kraft Pulp process, or after bleaching in a Kraft pulp process, or being added after repulping and before refining to a pulp slurry in the production of tissue, paper or paper board.

Although cellulolytic enzymes have been used successfully in commercial applications for many years, a need still exists for new cellulases with altered properties that make them particularly suitable for a desired purpose.

### SUMMARY OF THE INVENTION

The appended claims define the scope of protection. Any example and/or technical description of an apparatus, system, product and/or process in the description and/or drawing which is not covered by the claims is presented herein not as an embodiment of the invention but as an example useful for understanding the invention.

The inventors have surprisingly found that certain structural modifications can be made in an amino acid sequence of a cellulase and achieve a cellulase variant having cellulose degrading activity in liquid detergents. Additionally, some structural modifications to the cellulase were found to improve antigreying performance of the resulting variant in liquid detergents, when compared to the performance of the parent enzyme without said modifications.

According to a first aspect of the invention is provided a cellulase variant comprising an amino acid sequence having at least 80% sequence identity with the SEQ ID NO: 1 [A. strumarium Cel7], and at least one structural modification selected from: an amino acid substitution in the amino acid sequence of the cellulase; and a truncation of a loop defined by the amino acids 274-289.

The cellulase variant may have more than one of the above structural modifications. For example, it may have both a truncation or an absence of a loop, or a truncation or an absence of amino acid residues forming a loop in SEQ ID NO: 1, and additionally an amino acid substitution in the amino acid sequence of the cellulase.

In an embodiment the amino acid substitution is in at least one position selected from 37, 236, and 239. These positions have a role in substrate binding.

As evidenced by Examples below, the Cel7 cellulase (SEQ ID NO: 1) designated here as AS0, was successfully used as a parent enzyme (template cellulase) for designing and manufacturing various cellulase variants with structural modifications. Substitutions, insertions, and deletions could be successfully introduced in the Cel7 backbone in the positions specified herein, while retaining cellulolytic activity of the cellulase variant and allowing their production in host cells. Additionally, positions for structural modifications were identified that could be combined and that were shown to further improve antigreying performance of the cellulase variant.

In an embodiment the cellulase variant has an increased antigreying performance compared to a cellulase having the SEQ ID NO: 1. The antigreying performance can be determined as described in Example 4 below.

Further, the antigreying performance of the Cel7 cellulase without structural modification(s) was found to be increased, i.e. better, than that of commercially available cellulase enzymes used as a reference (see Example 4).

In an embodiment the cellulase comprises at least residues A37, D236, and A239. These residues were shown to be important for cellulase performance in detergent application tests, and they could be altered by substitutions resulting into different cellulase activity in an antigreying assay.

In an embodiment the cellulase comprises at least residues that correspond to the residues A37, D236, and A239 of SEQ ID NO: 1.

In an embodiment the structural modification is one or more substitution selected from 37S/T/N, 236S/P/G, and 239G/S.

In an embodiment the structural modification is one or more substitution selected from 37S/T/N, 236S/P/G, and 239G.

In an embodiment the structural modification is a substitution 37S. In another embodiment the structural modification is a substitution 37T. In another embodiment the structural modification is a substitution 37N.

In an embodiment the structural modification is a substitution 236S. In another embodiment the structural modification is a substitution 236P. In another embodiment the structural modification is a substitution 236G.

In an embodiment the structural modification is a substitution 239G. In another embodiment the structural modification is a substitution 239S.

In an embodiment the structural modification is a substitution of residues 236 and 239, preferably a substitution 236S/P/G and 239G/S, more preferably a substitution D236S/P/G and A239G/S.

In an embodiment the structural modification is one or more substitution selected from A37S/T/N, D236S/P/G, and A239G/S.

In an embodiment the structural modification is one or more substitution selected from A37S/T/N, D236S/P/G, and A239G.

In an embodiment the structural modification is a modification in one or two positions and selected from 37S/T/N, 236S, and 239G, more preferably from A37S/T/N, D236S/P, and A239G.

As the skilled person knows, a loop structure in a protein structure is a flexible and often unstructured segment within the three-dimensional structure of a protein. In the present cellulase the loop region defined by the amino acids 274-289 was shown to be important for cellulolytic activity in detergent applications, in particular to antigreying performance. In an embodiment the structural modification of the cellulase variant comprises a truncation of a loop defined by the amino acids 274-289, and the truncation comprises deleting one or more residues in the loop region 274-289 of the cellulase variant, thereby making the overall structure of the protein less flexible. In another embodiment the truncation comprises substituting of one or more residues in the region 274-289 by one or more amino acids. When deleting loop region residues, or when replacing loop region residues by a shorter amino acid chain, the truncation results in a variant having a smaller number of total amino acids compared to the parent.

For clarity, in the present disclosure amino acid sequences of variants having truncations or substitutions are discussed by referring to the original amino acid numbering of SEQ ID NO: 1.

In another embodiment a loop defined by the amino acids 274-287, 275-289, or 275-287 is truncated. In the present invention a truncation in such an amino acid region can be made in the same manner as specified elsewhere for the loop defined by the amino acids 274-289.

In another embodiment the truncation comprises deletion of one or more residues in the loop region 274-287 of the cellulase variant. In another embodiment the truncation comprises substituting one or more residues in the region 274-287 by one or more amino acids.

In another embodiment the truncation of a loop comprises replacing, substituting, or chemically modifying one or more residues in the region 274-289 by one or more residues, or chemically modifying such that the loop structure is changed. For example 14, 13, 12, 11, 10, 9, 8, 7, 6, 5, 4, 3, 2, or 1 residue(s) in the region 274-289 can be replaced by 1, 2, 3, 4, 5, or 6 residue(s). Preferably the region 274-289 is shortened by the replacement, i.e. the number of replaced residues is larger than the number of the replacing residues. Preferably, the replacement is a replacement of consecutive amino acids in the region 274-289.

In a preferred embodiment the residues 274-289 are replaced by 1, 2, 3, 4, or 5 residues. Preferably they are replaced by an amino acid sequence GG, GPG, NRRG (SEQ ID NO: 4), or NDTTG (SEQ ID NO: 5). In addition to the truncation of the loop, the cellulase variant may include a substitution of at least one of the residues 37 and 236, such as a substitution to 37S/T and/or 236S/P/G.

In a preferred embodiment the residues 276-289 are replaced by 1, 2, 3, 4, or 5 residues. Preferably they are replaced by amino acids GG, GPG, NRRG (SEQ ID NO: 4), or NDTTG (SEQ ID NO: 5), more preferably by GPG. In addition to the truncation of the loop, the cellulase variant may include a substitution of at least one of the residues 37 and 236, such as a substitution to 37S/T and/or 236S/P/G.

In a more preferred embodiment the residues 276-287 are replaced by 1, 2, 3, 4, or 5 residues. Preferably they are replaced by amino acids GG, GPG, NRRG (SEQ ID NO: 4), or NDTTG (SEQ ID NO: 5), more preferably by GPG. In addition to the truncation of the loop, the cellulase variant may include a substitution of at least one of the residues 37 and 236, such as a substitution to 37S/T and/or 236S/P/G.

In an embodiment the structural modification is a truncation of the loop defined by the amino acids 274-289, and the truncation is a substitution of the amino acids 274-289 by 1-5 amino acids, or by 1-4 amino acids.

In an embodiment the structural modification is a truncation of the loop defined by the amino acids 274-287, and the truncation is a substitution of the amino acids 274-287 by 1-5 amino acids, or by 1-4 amino acids.

In an embodiment the structural modification is a truncation of the loop defined by the amino acids 276-289, and the truncation is a substitution of the amino acids 276-289 by 1-5 amino acids, or by 1-4 amino acids.

In an embodiment the structural modification is a truncation of the loop defined by the amino acids 276-287, and the truncation is a substitution of the amino acids 276-287 by 1-5 amino acids, or by 1-4 amino acids.

In an embodiment the structural modification is a truncation of the loop as defined above, and additionally a substitution in one, two, or three positions 37, 236, and 239, such as a substitution 37T/S, 236S/P/G, and/or 239G.

In an embodiment the substitution is selected from (AS101) 274-287 replaced with NRRG (SEQ ID NO: 4), (AS102) 274-287 replaced with NDTTG (SEQ ID NO: 5), (AS103) 276-287 replaced with GG, and (AS104) 276-287 replaced with GPG. Additionally, the cellulase variant may include a substitution of at least one of the residues 37 and 236, such as a substitution to 37S/T and/or 236S/P/G.

In a preferred embodiment the substitution is selected from (AS101) S274-K287 replaced with NRRG (SEQ ID NO: 4), (AS102) S274-K287 replaced with NDTTG (SEQ ID NO: 5), (AS103) P276-K287 replaced with GG, and (AS104) P276-K287 replaced with GPG. Additionally, the cellulase variant may include a substitution of at least one of the residues 37 and 236, such as a substitution to 37S/T and/or 236S/P/G.

In an embodiment the present cellulase variant is varied by making at least one substitution in position 37, 236, and/or 239. One, two, or three substitutions are preferred.

In another aspect is provided a detergent comprising a cellulase enzyme having an amino acid sequence having at least 80% sequence identity with the SEQ ID NO: 1 and/or present cellulase variant, and at least one surfactant. It was surprisingly found that the present cellulase can be used in a detergent composition to an advantage and to provide a better antigreying performance compared to commercially available cellulases, and that the variants described herein provide additional advantages in such compositions and when compared to the parent enzyme or to the commercially available cellulases.

In another aspect is provided a method of manufacturing a detergent composition comprising providing a cellulase enzyme having an amino acid sequence having at least 80% sequence identity with the SEQ ID NO: 1 and/or the present cellulase variant, and at least one surfactant, and mixing to provide the detergent composition. The detergent composition can optionally be formulated to a desired formulation.

In an embodiment the detergent is provided in a form of a bar, a homogenous tablet, a tablet having two or more layers, a pouch having one or more compartments, a regular or compact powder, a granule, a paste, a gel, or a regular liquid, compact liquid, or concentrated liquid.

In an embodiment the detergent is a laundry detergent composition, preferably a liquid or solid laundry detergent composition.

In an embodiment the detergent further comprises other enzymes selected from the group of proteases, amylases, cellulases, lipases, xylanases, mannanases, cutinases, esterases, phosphodiesterases, phytases, DNases, pectinases, pectate lyases, pectinolytic enzymes, carbohydrases, arabinases, galactanases, xanthanases, xyloglucanases, laccases, peroxidases, and oxidases with or without a mediator.

In an embodiment the detergent further comprises at least one additive selected from the group of stabilizer, buffer, surfactant, builder, bleaching agent, mediator, anticorrosion agent, antiredeposition agent, caustic, abrasive, optical brightener, dye, pigment, and preservative.

In another aspect is provided a method of producing the present cellulase variant, comprising culturing a recombinant host cell having genetic elements configured to express a polypeptide having the amino acid sequence of the present cellulase variant, and recovering the polypeptide.

In another aspect is provided a method of producing the present cellulase enzyme, comprising culturing a recombinant host cell having genetic elements configured to express a polypeptide having at least 80% sequence identity with the SEQ ID NO: 1, and recovering the polypeptide.

According to another aspect is provided a method for antigreying, stain removal, fiber care, and color care, biostoning, or biofinishing a fabric made of cotton fibres or a fabric made of fibres of other cellulose based material,comprising a step of contacting the present cellulase variant or the present detergent with the cotton containing fabric.

In an embodiment the fibres of the other cellulose based materials include natural plant based fibres semi-synthetic fibres, fibres which can be derived from regenerated cellulose and are known e.g. as viscose, rayon, lyocell, modal, cupro or others, and fibres which are mostly produced from a wood based material.

According to another aspect is provided a method for antigreying, stain removal, fiber care, color care, biostoning, or biofinishing a cotton containing fabric, comprising a step of contacting the present cellulase, the present cellulase variant, or the present detergent, with the cotton containing fabric.

In an embodiment the cotton containing fabric is a garment, fabric, or yarn containing cotton.

According to another aspect is provided a method of generating a cellulase variant comprising the steps of:
(a) providing a template nucleic acid sequence of a template cellulase;
(b) identifying by biocomputing means active site residues and loop region residues of the template cellulase;
(c) generating variant nucleic acid sequences that encode candidate cellulase variants by modifying the template nucleic acid sequence by a modification, deletion, or addition of one or more amino acid in the active site residues and/or in loop region residues;
(d) expressing the template nucleic acid sequence in a recombinant host cell to provide template cellulase enzyme;
(e) expressing the variant nucleic acid sequences in a recombinant host cell to provide a candidate variant enzyme;
(f) testing and scoring the candidate variant enzymes, and using the template cellulase enzyme as a reference, for production level in the host cell, and antigreying performance; and
(g) selecting the candidate variant enzyme as the cellulase variant when the candidate variant enzyme reaches a predetermined scoring threshold value based on the performance in step (f).

In an embodiment, in the method of generating a cellulase variant, the template nucleic acid sequence of the template cellulase encodes SEQ ID NO: 1.

In an embodiment, in the method of generating a cellulase variant, the step (f) involves using a cellulase having the amino acid sequence SEQ ID NO: 1 as a reference to which the antigreying performance, and optionally the production level, is compared.

In an embodiment the predetermined scoring threshold level is selected from 5%, 10%, 15%, 20%, 25%, 30%, 50%, 75%, 100%, 150%, 200%, 250%, 300%, compared to the reference.

### FIGURES

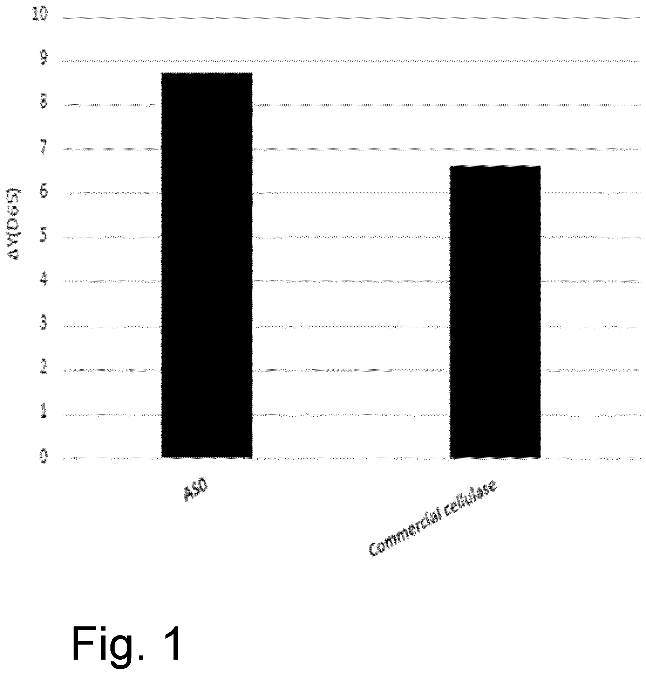

Figure 1 shows an antigreying performance of AS0 and commercial cellulase in commercial liquid detergent 1.

### DETAILED DESCRIPTION

Enzymes are catalytic proteins. Proteins are polypeptides. Polypeptides are long chains of amino acids linked by amide bonds. In the present disclosure, peptides are considered as being molecules composed of up to 20 amino acids, and polypeptides are molecules composed of more than 20 amino acids.

The generally accepted IUPAC single letter abbreviations for amino acids and their side chains in polypeptides are used herein.

In an embodiment the cellulase variant is a recombinant cellulase variant. The term recombinant means that it has been modified chemically and/or structurally from its native state, and/or produced by using recombinant technology or by gene manipulation technology. Recombinant proteins typically differ from a native sequence by one or more amino acids and/or because they are fused with one or more heterologous sequences.

The articles "a" and "an", as well as "another" and "further", are meant to refer to one or to more than one, that is to one or at least one, including several, of the grammatical object of "a", "an" or "another".

In an embodiment the present method of producing enzymes or the cellulase variant is an industrial scale method which optionally excludes for example analytical methods for research purposes.

In an embodiment any of the present methods is carried out in the sequence specified in a claim, aspect, or embodiment.

The word "comprise" and variations thereof such as "comprises" and "comprising" are meant inclusively and include additional possible components that are technically compatible as understood by a person skilled in the art. These terms also may in certain embodiments include their narrow meaning "consisting of".

Recitation of ranges of values herein are merely intended to serve as a way of referring individually to each separate value falling within the range, unless otherwise indicated. Each separate value is thus disclosed in the specification as if it were individually recited. In an embodiment, and as is understood by the skilled person in the context in which the expression is used, an open-ended range such as "less than 10" is to be construed as a range disclosing values below 10, but above 0. Such a range can be understood to include a lower limit of e.g. 0.0001, 0.001, 0.1, or 1 depending on the context. A lower limit of an open ended range can also be determined by the skilled person such that at least one technical effect is observable or measurable, thereby excluding insignificant trace amounts.

In embodiment the cellulase variant has cellulase activity. Cellulase activity means having cellulolytic activity that catalyzes the hydrolysis of 1,4-beta-D-glucosidic linkages in cellulose. Cellulolytic activity can be determined according to the procedure described in the Examples.

The term "parent" or "parent cellulase" or "template cellulase" means a polypeptide with cellulolytic activity to which a structural modification or alteration is made to produce the cellulase variant of the present invention. In a preferred embodiment the parent is a polypeptide having at least 80%, 85%, 90%, or 95% sequence identity with SEQ ID NO: 1, and structural modifications can be made in the parent sequence to obtain the present cellulase variants. Preferably the parent and the cellulase variant both have cellulolytic activity.

The present cellulase variant preferably comprises a sequence that is at least 80%, 81%, 82%, 83%, 84%, 85%, 86%, 87%, 88%, 89%, 90%, 91%, 92%, 93%, 94%, 95%, 96%, 97%, 98%, or at least 99%, but less than 100% identical to SEQ ID NO:1. In some embodiments, the cellulase variant sequence comprises at least one mutation or substitution. As used herein, "identity" means the percentage of exact matches of amino acid residues between two aligned sequences over the number of positions where there are residues present in both sequences. When one sequence has a residue with no corresponding residue in the other sequence, the alignment program allows a gap in the alignment, and that position is not counted in the denominator of the identity calculation. Identity is a value determined with the Pairwise Sequence Alignment 35 tool EMBOSS Needle at the EMBL-EBI website (www.ebi.ac.uk/Tools/psa/emboss needle/) with the following parameters: BLOSUM62, Gap open 10, Gap extend 0.5.

A cellulase variant according to the invention - and a gene encoding the enzyme according to the invention - can be derived from polypeptides and nucleic acids found in nature, or engineered from a synthetic polypeptide or synthetic nucleic acid with sequence information derived from nucleic acids or polypeptides found in nature. Such sequence information can be derived from more than one sequence found in nature, for example calculation of a common ancestor sequence, calculation of a synthetic sequence from an alignment of known homologous sequences, particularly calculation of the most frequent amino acid at each position and derivation of a consensus sequence.

Furthermore, a cellulase variant according to the invention - and a gene encoding the enzyme according to the invention - can contain one or more additional alterations or structural modification without a loss of function, or with a modified function. A functional enzyme can be verified for example by an enzyme activity measurement. Examples of such structural modifications and alterations are insertions, deletions and/or substitutions, preferably conservative substitutions, relative to a polypeptide or nucleic acid found in nature. The experimental exchangeability of amino acids in proteins has been reviewed by Yampolsky and Stoltzfus in Genetics 2005 pp 1459-1472 and can be applied to the enzymes used in the present invention.

Amino acid substitutions are designated herein by indicating their position and a single letter code separated by a slash, e.g. 50E/D means substitution in position 50 to E or D. Similarly, a substitution E50D means a substitution of E in position 50 to D. A substitution from E to D is an example of a conservative substitution, because E and D are both acidic amino acids that differ only in one methylene spacer in their side chains. Further examples of the conservative substitutions are substitutions within the group of basic amino acids (e.g. R/K/H), acidic amino acids and their amides (e.g. E/D/N/Q), aromatic amino acids (e.g. W/F/Y), hydrophobic amino acids (e.g. F/L/I/V/A/M), tiny amino acids (e.g. G/A/S), medium amino acids (e.g. T/S/A/V/C), charged amino acids (e.g. E/D/R/K/H) and other polar amino acids (e.g. S/T/N/Q/H). Beside substitutions by the twenty canonical amino acids, also other genetically encoded amino acids, for example selenocysteine and pyrrolysine, and so-called unnatural amino acids can be incorporated in proteins. By stepwise solid-phase peptide synthesis any available amino acid can be incorporated in peptides, which can be chemically ligated to larger peptides and polypeptides, to produce proteins and enzymes. Thus, in an embodiment a substitution in the cellulase variant is a conservative substitution, which substitution is likely to have only a small effect on the structure and/or function of the enzyme. In another embodiment the substitution is a non-conservative substitution, which may have a bigger effect on the structure and/or function of the enzyme.

An effect of a substitution to the present enzyme can be evaluated by computational means and experimentally by analysing structure and/or antigreying performance of the enzyme. The antigreying performance can be tested according to the washing test of Example 4, 5 or 6. The antigreying performance of the present enzymes could not be predicted base on the amino acid sequence, but required experimental analysis carried out according to the above Examples.

Furthermore, the present cellulase variant can be a fusion polypeptide in which another polypeptide and/or oligopeptide is fused to the amino-terminus and/or carboxyl-terminus of a polypeptide. Examples of such oligopeptides and polypeptides include poly histidine tags, signal peptides, linkers, binding domains, antibodies, chaperones, fluorescent proteins and enzymes.

The terms "depilling" (removal of pilling) and "color revival" are typically used to describe the cellulase variant's effects on old, used cotton textiles. The terms "antipilling" (prevention of pilling), "color maintenance" or "color care" are typically used to describe cellulase effects on new garments. The effect of fiber and color care properties can be detected by visible and measurable decrease of lightness (i.e. increase of darkness) or change in color of colored cotton textiles exposed to repeated washing cycles. Standardized tests monitors of prepilled and unpilled, new fabrics are commercially available.

The cellulase variant is especially useful in detergent applications as an antigreying agent. As used herein, the term "antigreying performance" or "antigreying effects" or "antigreying activity" mean antiredepositioning and pigment removal properties. With increasing number of wash cycles pigments, particles, soluble soils, salts, and other material can adhere on the textile fibers, most likely in those areas where cotton fibers are damaged. This can cause a greying effect of especially white fabrics and/or darkening or yellowing of white cotton textile. Without being bound by a theory, the cellulase variant hydrolyzes damaged cotton fibers at random positions of the cellulose polymer having an amorphous structure, resulting into a removal of fibers with attached particles or that have a higher accessibility for surfactants, and therefore providing a whitening or antigreying effect. In addition to the antigreying effect, traces of cellulosic material in the washing liquor may be digested, preventing the adhesion of such fibers on the cotton surface of the garment. These effects are called antigreying or antiredeposition, and they can be evaluated using optical measurements. Suitable test methods are generally known in the art and are typically based on using artificial ballast soil systems with standard white test fabrics in repeated washing cycles in washing machines. The antigreying effect can be tested also by a single wash as stressed test using redeposition liquid based on carbon black.

The cellulase variants may also be useful in finishing processes of the textile industry, such as biofinishing of fabrics, garments or yarn. As used in the present context, the expression "biofinishing" (also called depilling, defuzzing, dehairing or biopolishing) refers to the use of the variant enzymes in a controlled hydrolysis of cellulosic fibers in order to modify the fabric or yarn surface in a manner that permanently prevents the tendency for pilling, improves fabric handle like softness and smoothness, clears the surface structure by reducing fuzzing. Biofinishing results in clarification of colors, improves the drapability of the fabric and improves moisture absorbability, which may further improve also the dyeability. Biofinishing may be performed before, after, or at the same time as dyeing.

Enzymatic depilling can be carried out at any stage during textile wet processing, preferably after optional desizing and/or bleaching, and similar conditions as in biostoning can be used. Textiles in garment form can be also treated.

The cellulase variants may be used in biostoning of denim. As used in the present context, the expression "biostoning" of fabric or garment means the use of enzymes in place of, or in addition to, pumice stones for the treatment of fabric or garment, especially denim to obtain an aged or worn look. The term "aged or worn look" means that as a result of uneven dye removal, there are contrasts between dyed areas and areas from which dye has been removed.

The liquor ratio (the ratio of the volume of liquid per weight of fabric) in both biostoning and biofinishing may range from about 3:1 to 20:1, preferably 5:1 to 10:1. The treatment time can vary between 15 min to 90 min and preferably between 30 min to 60 min. The enzyme dosage depends on the type of the fabrics, machinery, process conditions (pH, temperature, liquor ratio, treatment time, denim load, process scale) and type of the enzyme preparation or composition. Typical process parameters for e.g. industrial biofinishing are pH 4.5 - 8 at temperature of 40 - 65°C. A person skilled in art is capable in defining suitable dosages and conditions when using the present cellulase variant.

In other applications that employ the cellulolytic or antigreying performance of the present cellulase variant, it is preferred to use an effective amount of the cellulase variant. The skilled person is capable of determining a suitable effective amount depending on the application and taking into account the process conditions and the formulation in which the cellulase variant is provided, including the specific activity of the cellulase variant in the formulation.

When the present cellulase variant is used to degrade cellulose, cellulosic material is reacted with the present cellulase variant under suitable conditions, such as appropriate pH, and temperature, and the reaction is allowed to continue for a time sufficient for the cellulolytic reaction to take place, whereby at least partially hydrolyzed cellulosic material is obtained.

The cellulase variant may be added to detergent compositions to improve textile cleaning, for instance by removal of pigment dirt and by antiredeposition and antigreying. The cellulase variant may also improve fiber and color care properties by prevention and removal of fuzz and pills resulting in brightening or freshening of colors and softening.

In detergents, the amount of the cellulase variant can vary depending on the desired amount of cellulase activity.

A detergent for use as a solid laundry detergent, may include 0.000001 % - 5%, such as 0.000005-2%, such as 0.00001%-1%, such as 0.00001%-0.1% of variant cellulase polypeptide by dry weight of the composition.

A detergent for use as a laundry liquid, may include 0.000001%-3%, such as 0.000005%-1 %, such as 0.00001%-0.1% of variant cellulase polypeptide by weight of the composition.

A detergent for use in automatic dishwash, may include 0.000001%-5%, such as 0.000005%-2%, such as 0.00001%-1%, such as 0.00001%-0.1% of variant cellulase polypeptide by weight of the composition.

In an embodiment the detergent is in the form of a bar, a homogenous tablet, a tablet having two or more layers, a pouch having one or more compartments, a regular or compact powder, a granule, a paste, a gel, or a regular, compact, or concentrated liquid. In one embodiment the detergent is a laundry detergent composition, preferably a liquid or solid laundry detergent composition.

The cellulase variant and/or the detergent may be used for treating any cellulosic material. In the present context, "cellulosic material" refers to any material comprising cellulose or derivatives thereof as a significant component. Such a material may be textile material, plants or material of plant origin used in food or animal feed, plant material for oil extraction, or wood-derived mechanical or chemical pulp or secondary fiber. The use or a method involving such use comprises contacting the material to be treated with an effective amount of the present variant or detergent.

In an embodiment the present cellulase variant is used in a method of making paper or paper board, wherein the method comprises: providing a cellulase composition comprising: cellulase; adding the cellulase composition to a pulp slurry to produce a treated pulp slurry; and refining the treated pulp slurry to a desired freeness; and forming a paper or paperboard.

In an embodiment the present cellulase variant is used in method of producing dissolving pulp comprising the steps of: i) treating unbleached, or partially bleached, or alkaline extracted dissolving pulp, with one or more cellulases (X stage); and ii) bleaching of unbleached/partially bleached/alkaline extracted pulp; and iii) optionally performing alkaline extraction of unbleached/partially bleached/alkaline extracted pulp; and thereby generating dissolving pulp with reduced viscosity.

In the above method the cellulase is preferably the present cellulase variant or detergent.

In an embodiment the variant and/or the detergent is used to treat wood-derived pulp, chemical pulp, paper, paperboard, or secondary fiber.

Preferably the present cellulase variant has an improved antigreying performance compared to the parent Cel7 cellulase. The improved antigreying performance can be analysed and confirmed by the method of Example 4, 5 and 6.

In an embodiment the antigreying performance can be analysed by washing cotton swatches with liquid detergent in a redeposition liquid based on carbon black in the presence of a cellulase variant, measuring reflectance of the swatches after the wash, and calculating antigreying performance of the cellulase variant based on a reflectance difference to a washing without the cellulase variant. The improved antigreying performance of the variant can be verified by comparing to the reflectance difference obtained by using a reference enzyme such as the parent enzyme Cel7 from which the present cellulase variants derived.

Some further embodiments are described below:
Embodiment 1. A cellulase variant comprising an amino acid sequence having at least 80% sequence identity with the SEQ ID NO: 1, and at least one structural modification selected from; a truncation of a loop defined by the amino acids 274-289; at least a partial absence of amino acids corresponding to positions 274-289; and an amino acid substitution in the amino acid sequence of the cellulase.
Embodiment 2. The cellulase variant of embodiment 1, wherein the structural modification is one or more substitution selected from 37S/T/N, 236S/P/G, and 239G/S.
Embodiment 3. The cellulase variant of embodiment 1 or 2, wherein the structural modification is one or more substation selected from A37S/T/N, D236S/P/G, and A239G/S.
Embodiment 4. The cellulase variant of embodiment 2 or 3, wherein the structural modification is a modification in one or two positions selected from 37S/T/N, 236S/P, and 239G, more preferably from A37S/T/N, D236S/P, and A239G.
Embodiment 5. The cellulase variant of any one of the embodiments 2-4 having an increased antigreying performance

### SEQUENCE LISTINGS

SEQ ID NO: 1 is an amino acid sequence of mature AS0 cellulase without signal sequence, 415 amino acids.
SEQ ID NO: 2 is a nucleotide sequence encoding the amino acid sequence of mature AS0 cellulase, 1248 bp.
SEQ ID NO: 3 is an amino acid sequence of the full-length 50K *Melanocarpus albomyces* cellulase.
SEQ ID NO: 4 is an amino acid sequence NRRG.
SEQ ID NO: 5 is an amino acid sequence NDTTG.
SEQ ID NO: 6 is an amino acid sequence STPNPPQNGKGHNK.
SEQ ID NO: 7 is an amino acid sequence PNPPQNGKGHNK.

### EXAMPLES

### Variant design

The cellulase variant or mutant endoglucanase of the present invention was designed based on sequence and structure comparison of the Endoglucanase I (Cel7B), Humicola insolens 2A39_A and Talaromyces emersonii 6SU8_1. Also, amino acid sequences of other endoglucanases I were used in comparisons.

A change in the amino acid sequence of the cellulase variant may be obtained by constructing a modified nucleotide sequence or DNA sequence by using genetic engineering. As a result, a modified nucleotide sequence is obtained, which encodes the variant or mutant polypeptide of the invention. The methods for modifying the nucleotide sequences include e.g. site-directed and random mutagenesis. For site-directed mutagenesis, a method based on protein structure, a good understanding of the structure-function relationship would be beneficial. In the absence of such deep understanding, methods based on random mutagenesis may be used. For example, as disclosed in Sambrook and Russell (2001) oligonucleotide-directed mutagenesis for changing the base sequence of a segment of the coding DNA may be used to test the role of residues in the structure and catalytic activity of a protein. In the absence of a three-dimensional structure, this type of protein engineering relies on educated guesses concerning the structure of the protein and contribution of individual residues to protein stability and function.

Specific examples of cellulase variants according to the present invention are described herein. The skilled person understands that the specific examples are merely illustrative examples of structural modifications that can be made to make cellulase variants. The examples below provide methods that the skilled person can use to manufacture and identify substitution sites that provide effective cellulase variants.

Standard molecular biology methods were used in the isolation and enzyme treatments of DNA (e.g. isolation of plasmid DNA, digestion of DNA to produce DNA fragments), in *E. coli* transformations, sequencing etc. The basic laboratory methods used were either as described by the enzyme, reagent or kit manufacturer or as described in the standard molecular biology handbooks, e.g. Sambrook and Russell (2001) or as described in the following examples.

### Example 1. Backbone screening/ Bio IT sequence search

In order to identify novel cellulase candidates a protein family analysis was conducted. The Glycoside Hydrolase Family 7 (GH7 family) was chosen for the search.

To start the protein family analysis of GH7, all proteins belonging to the protein family were collected. Proteins were retrieved by homology-based BLAST searches and by protein motif search. The query sequence in the BLAST searches was 50K (SEQ ID NO: 3).

The BLAST searches were conducted against several sequence databases, to get maximum coverage of the available sequences. The following databases were queried using command line BLAST: NCBI protein databases (nr, pdbaa), NCBI nucleotide databases (nt, env_nt), Uniprot protein database (including both SwissProt and TrEMBL), proprietary genomes of fungal and bacterial species, and patent sequence data via SequenceBase i.e. protein sequences from Thomson Reuters GENESEQ^{™}, USGENE^{®} , WOGENE.

The BLAST searches were conducted using a shell script in a Linux environment. The script goes through the databases and for each database conducts a batch BLAST search using all query proteins sequences simultaneously. E-value threshold of 1e-30 was used. The BLAST was requested to return 10000 best hits for each query. Otherwise default parameters were used. The script then retrieved the DNA/AA sequence of matching hits. For BLAST hits from nucleotide databases a specially built script was used to process the xml-format BLAST output and extract the matching portion as an amino acid sequence. Glycoside Hydrolase Family 7 proteins in *Sordariales* genomes were downloaded from the Joint Genome Institute website (https://mycocosm.jgi.doe.gov/).

After retrieval of the candidate sequences, a phylogenetic tree was constructed to get an understanding of the diversity among the retrieved sequence. The construction of the phylogenetic tree consisted of clustering of sequences to remove redundancy, followed by multiple sequence alignment and building of the phylogenetic tree.

The protein sequences were clustered before the multiple sequence alignment and phylogenetic tree construction. This was done to remove the redundancy. To get an even coverage of the sequence space, the sequences were clustered to 95% identity using the CD-HIT clustering program ((http://weizhongli-lab.org/cd-hit/).

The selected candidates were aligned using MAFFT (https://mafft.cbrc.ip/aiignment/software/), a multiple sequence alignment program. A phylogenetic tree was built based on the multiple sequence alignment using FastTree (http://www.microbesoniine.orq/fasttree/) algorithm. FastTree infers approximately-maximum-likelihood phylogenetic trees from alignments of nucleotide or protein sequences.

Candidate sequences for expression were selected from the branch containing the 50K cellulase. This branch had 474 sequences of fungal origin. Altogether 20 sequences were selected for testing.

20 genes were expressed in *T. reesei* (Example 2). Expression levels of recombinant enzymes were determined by measuring NCU activity (Example2). Enzyme application performance was evaluated (Example 4). *Achaetomium strumarium* (SEQ ID NO: 1) cellulase was selected for further enzyme engineering.

### Example 2. Production of Achaetomium strumarium Endoglucanase I in Trichoderma reesei

The cellulase gene, designated here as AS0 (nucleic acid sequence SEQ ID NO: 2, corresponding to amino acid sequence SEQ ID NO: 1), encoding *Achaetomium strumarium* Endoglucanase I, was ordered as synthetic gene with *Trichoderma reesei* codon usage.

For production of recombinant AS0, the gene was fused (direct fusion) to *T. reesei cbh1* (cellobiohydrolase 1) promoter in expression cassette. The transcription was terminated by the *T. reesei cbh2* (cellobiohydrolases 2) terminator. Synthetic gene encoding acetamidase (AmdS) was used as a transformation marker in the plasmid as described in Paloheimo *etal.* 2003. In addition, the expression plasmid contains *cbh1* 3' and 5' flanking regions for optionally targeting the expression vector into the *cbh1* locus.

The plasmid including the expression cassette was transformed to *T. reesei* using protoplast transformation. The transformants were selected on plates containing acetamide as the sole nitrogen source. The *T. reesei* host strain used is lacking four main endogenous cellulases: CBHI/Cel7A, CBHII/Cel6A, EGI/Cel7B and EGII/Cel5A. The transformations were performed according to (Penttilä et al., 1987), with the modifications described in (Karhunen et al., 1993). Additionally, CRISPR-CAS technology can be used in transformations (Rantasalo, A. et al., 2019).

Transformants were purified on selection plates through single conidia prior to sporulating them on potato dextrose agar (PDA). The endoglucanase production of the transformants was analyzed from the culture supernatants of shake flask cultivations (50 mL). Transformants were grown for 7 days at 30°C, 250rpm in a complex cellulase-inducing medium (Joutsjoki et al., 1993) buffered with 5 % (w/v) KH₂PO₄ at pH 5.5 to obtain material for the application tests (Example 4).

The enzyme activity of the recombinant protein was measured from the culture supernatant as the release of reducing sugars from carboxymethylcellulose (3% CMC) at 50°C in 50mM HEPES buffer pH 7.0 essentially as described by Bailey and Nevalainen, 1981; Haakana, *et al,* 2004 (NCU activity). Production of the recombinant protein was also detected from the culture supernatant by sodium dodecyl sulfate polyacrylamide gel electrophoresis (SDS-PAGE).

### Example 3. Production of Achaetomium strumarium AS variants in Trichoderma reesei

Standard molecular biology methods were used as described in Example 2. The parental molecule for construction of *Achaetomium strumarium* Endoglucanase I variants is the wild type cellulase AS0 (nucleic acid sequence SEQ ID NO: 2, corresponding to amino acid sequence SEQ ID NO: 1). The endoglucanase genes encoding the designed endoglucanase variants (Table 1) were ordered as synthetic genes with *Trichoderma reesei* codon usage. Expression plasmids were constructed, and recombinant proteins produced as described in Example 2.

**Table 1. The synthetic genes used in construction of the expression cassettes for production of AS endoglucanase variants in T. reesei. Amino acid positions are numbered by correspondence with the mature amino acid sequence without signal sequence (SEQ ID NO: 1).**

| **Variant code** | **Amino acid change in parent AS0 cellulase** | **Variant structural modification** |
|---|---|---|
| AS45 | A37T | 37T |
| AS47 | A37N | 37N |
| AS51 | A37S, P216K | 37S, 216K |
| AS52 | A37S, V389R | 37S, 389R |
| AS53 | A37S, N58D, V389R | 37S, 58D, 389R |
| AS57 | D236S | 236S |
| AS58 | D236P, A239G | 236P, 239G |
| AS59 | D236G | 236G |
| AS60 | P216K, T262R | 216K, 262R |
| AS62 | P216K, T262R, V389R | 216K, 262R, 389R |
| AS64 | N281K, D311R, T396K | 281K, 311R, 396K |
| AS65 | N281K, K289R, T396K | 281K, 289R, 396K |
| AS66 | N281K, K289R, D311R | 281K, 289R, 311R |
| AS73 | A37S, A239S | 37S, 239S |
| AS83 | A37S, D236S | 37S, 236S |
| AS84 | A37S, D236P | 37S, 236P |
| AS85 | A37S, D236G | 37S, 236G |
| AS86 | A37T, D236S | 37T, 236S |
| AS87 | A37T, D236P | 37T, 236P |
| AS88 | A37T, D236G | 37T, 236G |
| AS89 | A37N, D236S | 37N,236S |
| AS90 | A37N, D236P | 37N, 236P |
| AS91 | A37N, D236G | 37N, 236G |
| AS92 | A37S, D236S, A239G | 37S, 236S, 239G |
| AS93 | A37S, D236P, A239G | 37S, 236P, 239G |
| AS94 | A37S, D236G, A239G | 37S, 236G, 239G |
| AS95 | A37T, D236S, A239G | 37T, 236S, 239G |
| AS96 | A37T, D236P, A239G | 37T, 236P, 239G |
| AS97 | A37T, D236G, A239G | 37T, 236G, 239G |
| AS98 | A37N, D236S, A239G | 37N, 236S, 239G |
| AS99 | A37N, D236P, A239G | 37N, 236P, 239G |
| AS100 | A37N, D236G, A239G | 37N, 236G, 239G |
| AS101 | S274-K287 STPNPPQNGKGHNK (SEQ ID NO: 6)) replaced with NRRG (SEQ ID NO: 4) | 274-287 replaced with NRRG (SEQ ID NO: 4) |
| AS102 | S274-K287 (STPNPPQNGKGHNK (SEQ ID NO: 6)) replaced with NDTTG (SEQ ID NO: 5) | 274-287 replaced with NDTTG (SEQ ID NO: 5) |
| AS103 | P276-K287 (PNPPQNGKGHNK (SEQ ID NO: 7)) replaced with GG | 276-287 replaced with GG |
| AS104 | P276-K287 (PNPPQNGKGHNK (SEQ ID NO: 7)) replaced with GPG | 276-287 replaced with GPG |
| AS105 | A37T, D236S, loop deletion [P276-K287 replaced with GPG] | 37T, 236S, loop deletion [276-287 replaced with GPG] |
| AS106 | A37T, D236S, loop deletion [P276-K287 replaced with GPG], LCBM | 37T, 236S, loop deletion [276-287 replaced with GPG], LCBM |
| AS107 | loop deletion [P276-K287 replaced with GPG], LCBM | loop deletion [276-287 replaced with GPG], LCBM |
| AS108 | A37S, D236S, loop deletion [P276-K287 replaced with GPG], LCBM-Ins | 37S, 236S, loop deletion [276-287 replaced with GPG], LCBM-Ins |
| AS109 | A37S, D236S, loop deletion [P276-K287 replaced with GPG], LCBM-Ins-3MUT | 37S, 236S, loop deletion [276-287 replaced with GPG], LCBM-Ins-3MUT |

### Example 4. Testing the antigreying performance of AS0 in Launder-Ometer in commercial liquid detergent 1

AS0 produced in *Trichoderma* as described in Example 2 was tested for antigreying performance by a single wash method (40°C, 60 min, 16°dH) using carbon black (approx. 0.15g/l) in a wash solution in addition to detergent (3.2 g/l). Commercial cellulase was used as reference.

Cotton interlock double jersey with optical brighteners (CN-42) supplied from CFT (Center for Testmaterials NV, the Netherlands) was used as test fabric. The fabric was first prewashed in a washing machine (15 min 50°C and 60 min at 60°C) and tumble dried, then cut to swatches of approx. 14x14.5cm (total weight of 4 swatches 25g).

As a source of carbon black RD-liq 01 from CFT containing 7 g of carbon black liquid (about 33% carbon black particles) in plastic bottles, that are normally intended for full scale washes in washing machine (one bottle per one single wash with test fabrics), were used. In this Example the method was adapted to small scale using approximately similar ratio of carbon black and water that would be in full scale. First a stock solution carbon black was prepared by placing an opened bottle of RD-liq 01 (i.e. about 2.3g carbon black particles) in a decanter flask containing 1 liter of deionized water. The solution was stirred with a magnetic stirrer for overnight until the contents of the bottle were totally released. After that 65 g of stock solution mixed with 935ml of synthetic tap water with hardness of 17.1°dH ending up to diluted carbon black solution having hardness of 16°dH and carbon black content approximately 0.15g/l (or 0.45g RD-liq 01).

For synthetic tap water with hardness of 17.1°dH the following stock solutions were prepared in deionized water (Milli-Q or equivalent):
Stock solution with 1000°d Calcium-hardness: CaCl₂ x 2 H₂O (1.02382.1000, Merck KgaA, Germany) 26.22g/l
Stock solution with 200°d Magnesium-hardness: MgSO₄ x 7 H₂O (1.05886.1000, Merck KgaA, Germany) 8.79g/l H₂O

NaHCOs stock solution: NaHCOs (1.06329.1000 Merck KgaA, Germany) 29.6g/l.

14.2ml CaCl₂ solution, 14.2ml MgSO₄ solution and 10.0ml of freshly made NaHCO₃ solution were added in volumetric flask in the given order, made up to 1 liter with deionized water and mixed.

Antigreying tests were performed in Atlas LP-2 Launder-Ometer as follows. Launder-Ometer was first preheated to 40°C. 60g of steel balls (diameter 0.6 cm), 0.79g of commercial liquid detergent described in Table 2, 250ml of diluted carbon black solution having hardness of 16°dH and diluted enzyme (<1.0ml) were added into 1.2 liter containers. After that, 4 swatches of prewashed test fabric CN-42 were added and the Launder-Ometer was run at 40°C for 60min with a rotation speed of 42rpm. Enzymes were dosed 0.2 activity units (NCU) per liter.

**Table 2. Composition of commercial liquid detergent 1**

| Ingredient | (%) |
|---|---|
| Anionic surfactants | 15 - 30 |
| Nonionic surfactants, soap | 5 - 15 |
| Boric acid | 1 - 2.5 |
| Other ingredients: e.g. citric acid, builder, polyols, solvents, optical brighteners, perfumes, preservatives | |
| Water | to 100% |
| pH | 7.5-8.5 |

After the cellulase treatment in Launder-Ometer, the swatches were first quickly rinsed separately under running tap water (ca. 20°C) to remove the steel balls, then rinsed separately under running water in specific cups containing holes for 3 times and finally dipped in a bucket containing water. After that the swatches were spin-dried in a washing machine and let to dry on a grid at room temperature. Enzyme treated fabrics and controls without enzyme were rinsed and spin-dried separately to avoid contamination.

Antigreying performance of cellulase was evaluated by measuring reflectance of test fabrics by Konica Minolta CM3610A spectrophotometer as Y-value (illuminant D65/10°, 420nm cut). Cellulase performance was calculated as ΔY (delta Y), which means value Y of enzyme treated fabric minus value Y of fabric treated with carbon black and detergent containing washing liquor without enzyme (enzyme blank, control). Values were the average of 4 swatches. The higher the Y or ΔY value, the better the antigreying effect and whiteness of the fabric.

Results in Figure 1 show that AS0 is a cellulase with surprisingly good antigreying performance. Figure 1 shows that AS0 has higher antigreying performance compared to that of commercial cellulase.

### Example 5. Screening the antigreying performance of AS0 variants AS45-AS59, AS73 in Launder-Ometer in commercial liquid detergent 1

AS0 variants (AS45-AS59, AS73) produced in *Trichoderma* as described in Example 3 were tested for antigreying performance by a single wash method (40°C, 60 min, 16°dH) using carbon black (approx. 0.15g/l) in a wash solution in addition to detergent (3.2 g/l). Parental cellulase AS0 was used as reference.

Cotton interlock double jersey with optical brighteners (CN-42) supplied from CFT (Center for Testmaterials NV, the Netherlands) was used as test fabric. The fabric was first prewashed in a washing machine (15min 50°C and 60min at 60°C) and tumble dried, then cut to swatches of approx. 14x14.5cm (total weight of 4 swatches 25g).

As a source of carbon black RD-liq 01 from CFT containing 7 g of carbon black liquid (about 33 % carbon black particles) in plastic bottles, that are normally intended for full scale washes in washing machine (one bottle per one single wash with test fabrics), were used. In this Example the method was adapted to small scale using approximately similar ratio of carbon black and water that would be in full scale. First a stock solution carbon black was prepared by placing an opened bottle of RD- liq 01 (i.e. about 2.3g carbon black particles) in a decanter flask containing 1 liter of deionized water. The solution was stirred with a magnetic stirrer for overnight until the contents of the bottle were totally released. After that 65g of stock solution mixed with 935 ml of synthetic tap water with hardness of 17.1°dH ending up to diluted carbon black solution having hardness of 16°dH and carbon black content approximately 0.15g/l (or 0.45 g RD-liq 01).

For synthetic tap water with hardness of 17.1°dH the following stock solutions were prepared in deionized water (Milli-Q or equivalent):
Stock solution with 1000°d Calcium-hardness: CaCl₂ x 2 H₂O (1.02382.1000, Merck KgaA, Germany) 26.22 g/l
Stock solution with 200°d Magnesium-hardness: MgSO₄ x 7 H₂O (1.05886.1000, Merck KgaA, Germany) 8.79 g/l H₂O

NaHCOs stock solution: NaHCOs (1.06329.1000 Merck KgaA, Germany) 29.6g/l.

14.2ml CaCl₂ solution, 14.2ml MgSO₄ solution and 10.0ml of freshly made NaHCO₃ solution were added in volumetric flask in the given order, made up to 1 liter with deionized water and mixed.

Antigreying tests were performed in Atlas LP-2 Launder-Ometer as follows. Launder-Ometer was first preheated to 40°C. 60g of steel balls (diameter 0.6cm), 0.79g of commercial liquid detergent described in Table 2, 250ml of diluted carbon black solution having hardness of 16°dH and diluted enzyme (<1.0ml) were added into 1.2 liter containers. After that, 4 swatches of prewashed test fabric CN-42 were added and the Launder-Ometer was run at 40°C for 60min with a rotation speed of 42rpm. Enzymes were dosed 0.2 or 0.6 activity units (NCU) per liter.

After the cellulase treatment in Launder-Ometer, the swatches were first quickly rinsed separately under running tap water (ca. 20°C) to remove the steel balls, then rinsed separately under running water in specific cups containing holes for 3 times and finally dipped in a bucket containing water. After that the swatches were spin-dried in a washing machine and let to dry on a grid at room temperature. Enzyme treated fabrics and controls without enzyme were rinsed and spin-dried separately to avoid contamination.

Antigreying performance of cellulase was evaluated by measuring reflectance of test fabrics by Konica Minolta CM3610A spectrophotometer as Y-value (illuminant D65/10°, 420nm cut). Cellulase performance was calculated as ΔY (delta Y), which means value Y of enzyme treated fabric minus value Y of fabric treated with carbon black and detergent containing washing liquor without enzyme (enzyme blank, control). Values were the average of 4 swatches. The higher the Y or ΔY value, the better the antigreying effect and whiteness of the fabric.

Results in Table 3 show that the tested variants have at least as good (0) antigreying performance compared to parental cellulase AS0 (data not shown). AS58 has better performance (+) compared to AS0, when tested with commercial detergent 1.

**Table 3. Antigreying performance of the variants in commercial liquid detergent 1**

| **Variant code** | **Performance** |
|---|---|
| AS45 | 0 |
| AS47 | 0 |
| AS51 | 0 |
| AS52 | 0 |
| AS53 | 0 |
| AS57 | 0 |
| AS58 | + |
| AS59 | 0 |
| AS73 | 0 |

### Example 6. Testing the antigreying performance of AS variants in Launder-Ometer in commercial detergent 2

AS0 variants (AS45, AS58, AS83-AS113) produced in *Trichoderma* as described in Example 3 were tested for antigreying performance by a single wash method (40°C, 60min, 16°dH) using carbon black (approx. 0.15g/l) in a wash solution in addition to detergent (3.1g/l). Parental cellulase AS0 was used as reference.

Cotton interlock double jersey with optical brighteners (CN-42) or cotton interlock double jersey with optical brighteners, pre-washed 3x with IEC A* (CN-42 (w3x)) supplied from CFT (Center for Testmaterials NV, the Netherlands) was used as test fabric. CN-42 fabric was prewashed in a washing machine (similar as described in Example 4 or 3 x 1h 40 min 90°C with W-IEC A* reference base detergent without phosphate and 1h 40 min 90°C) and tumble dried. Prewashed CN-42 fabric and CN-42(w3x) fabric were cut to swatches of approx. 13.5-14x14.5-16 cm (total weight of 4 swatches 25g).

As a source of carbon black RD-liq 01 from CFT containing 7g of carbon black liquid (about 33% carbon black particles) in plastic bottles, that are normally intended for full scale washes in washing machine (one bottle per one single wash with test fabrics), were used. In this Example the method was adapted to small scale using approximately similar ratio of carbon black and water that would be in full scale. First a stock solution carbon black was prepared by placing an opened bottle of RD-liq 01 (i.e. about 2.3g carbon black particles) in a decanter flask containing 1 liter of deionized water. The solution was stirred with a magnetic stirrer for overnight until the contents of the bottle were totally released. After that 65g of stock solution mixed with 935ml of synthetic tap water with hardness of 17.1°dH ending up to diluted carbon black solution having hardness of 16°dH and carbon black content approximately 0.15g/l (or 0.45g RD-liq 01).

For synthetic tap water with hardness of 17.1°dH the following stock solutions were prepared in deionized water (Milli-Q or equivalent):
Stock solution with 1000°d Calcium-hardness: CaCl₂ x 2 H₂O (1.02382.1000, Merck KgaA, Germany) 26.22g/l
Stock solution with 200°d Magnesium-hardness: MgSO₄ x 7 H₂O (1.05886.1000, Merck KgaA, Germany) 8.79 g/l H₂O

NaHCOs stock solution: NaHCOs (1.06329.1000 Merck KgaA, Germany) 29.6g/l.

14.2ml CaCl₂ solution, 14.2ml MgSO₄ solution and 10.0ml of freshly made NaHCO₃ solution were added in volumetric flask in the given order, made up to 1 liter with deionized water and mixed.

Antigreying tests were performed in Atlas LP-2 Launder-Ometer as follows. Launder-Ometer was first preheated to 40°C. 60g of steel balls (diameter 0.6cm), 0.77g of commercial liquid detergent described in Table 4, 250ml of diluted carbon black solution having hardness of 16°dH and diluted enzyme (<1.0ml) were added into 1.2 liter containers. After that, 4 swatches of prewashed test fabric CN-42 or test fabric CN-42(w3x) were added and the Launder-Ometer was run at 40°C for 60min with a rotation speed of 42rpm. Enzymes were dosed 0.1, 0.2 or 0.6 activity units (NCU) per liter.

**Table 4. Composition of commercial liquid detergent 2**

| Ingredient | % |
|---|---|
| Anionic surfactants | 5 - 15 |
| Nonionic surfactants, soap | 5 - 15 |
| Boric acid | ≤ 0,2 |
| Other ingredients: e.g. citric acid, builder, polyols, solvents, optical brighteners, perfumes, preservatives | |
| water | to 100 % |
| pH | 7.5-8.5 |

After the cellulase treatment in Launder-Ometer, the swatches were first quickly rinsed separately under running tap water (ca. 20°C) to remove the steel balls, then rinsed separately under running water in specific cups containing holes for 3 times and finally dipped in a bucket containing water. After that the swatches were spin-dried in a washing machine and let to dry on a grid at room temperature. Enzyme treated fabrics and controls without enzyme were rinsed and spin-dried separately to avoid contamination.

Antigreying performance of cellulase was evaluated by measuring reflectance of test fabrics by Konica Minolta CM3610A spectrophotometer as Y-value (illuminant D65/10°, 420 nm cut). Cellulase performance was calculated as ΔY (delta Y), which means value Y of enzyme treated fabric minus value Y of fabric treated with carbon black and detergent containing washing liquor without enzyme (enzyme blank, control). Values were the average of 4 swatches. The higher the Y or ΔY value, the better the antigreying effect and whiteness of the fabric.

Results in Table 5 show that the tested variants could be successfully produced as an active enzyme and they have at least as good (0) antigreying performance compared to parental cellulase AS0 (data not shown). AS58, AS93, AS95 and AS98 have better performance (+) compared to AS0. Variants AS101-AS113, which belong to the group of loop truncated AS variants, have clearly improved (++) or highly improved (+++) antigreying performance compared to parental cellulase AS0 when tested with commercial liquid detergent 2.

**Table 5. Antigreying performance of the variants in commercial liquid detergent 2**

| **Variant code** | **Performance** |
|---|---|
| AS45 | 0 |
| AS58 | + |
| AS83 | 0 |
| AS84 | 0 |
| AS85 | 0 |
| AS86 | 0 |
| AS87 | 0 |
| AS88 | 0 |
| AS89 | 0 |
| AS90 | 0 |
| AS91 | 0 |
| AS92 | 0 |
| AS93 | + |
| AS94 | 0 |
| AS95 | + |
| AS96 | 0 |
| AS97 | 0 |
| AS98 | + |
| AS99 | 0 |
| AS100 | 0 |
| AS101 | ++ |
| AS102 | ++ |
| AS103 | +++ |
| AS104 | +++ |
| AS105 | +++ |

The foregoing description has provided by way of non-limiting examples of particular implementations and embodiments a full and informative description of the best mode presently contemplated by the inventors for carrying out the invention. It is however clear to a person skilled in the art that the invention is not restricted to details of the embodiments presented in the foregoing, but that it can be implemented in other embodiments using equivalent means or in different combinations of embodiments without deviating from the characteristics of the invention.

Furthermore, some of the features of the afore-disclosed example embodiments may be used to advantage without the corresponding use of other features. As such, the foregoing description shall be considered as merely illustrative of the principles of the present invention, and not in limitation thereof. Hence, the scope of the invention is only restricted by the appended patent claims.

## Claims

1. A cellulase variant comprising an amino acid sequence having at least 80% sequence identity with the SEQ ID NO: 1, and at least one structural modification selected from;
a truncation of a loop defined by the amino acids 274-289;
at least a partial absence of amino acids corresponding to positions 274-289; and
an amino acid substitution in the amino acid sequence of the cellulase.

2. The cellulase variant of claim 1, wherein the structural modification is one or more substitution selected from
37S/T/N,
236S/P/G, and
239G/S.

3. The cellulase variant of claim 1 or 2, wherein the structural modification is one or more substitution selected from
A37S/T/N,
D236S/P/G, and
A239G/S.

4. The cellulase variant of claim 2 or 3, wherein the structural modification is a modification in one or two positions selected from 37S/T/N, 236S/P, and 239G, more preferably from A37S/T/N, D236S/P, and A239G.

5. The cellulase variant of any one of claims 2-4 having an increased antigreying performance compared to a cellulase having the SEQ ID NO: 1.

6. The cellulase variant of claim 1, wherein the structural modification is a truncation of the loop defined by the amino acids 274-289, and wherein the truncation is a substitution of amino acids 274-289 by 1-4 amino acids.

7. The cellulase variant of claim 6, wherein the substitution is selected from (AS101) S274-K287 replaced with NRRG (SEQ ID NO: 4), (AS102) S274-K287 replaced with NDTTG (SEQ ID NO: 5), (AS103) P276-K287 replaced with GG, and (AS104) P276-K287 replaced with GPG.

8. A detergent comprising a cellulase enzyme having an amino acid sequence having at least 80% sequence identity with the SEQ ID NO: 1 and/or the cellulase variant of any one of the claims 1-7, and at least one surfactant.

9. The detergent of claim 8, wherein the detergent is provided in a form of a bar, a homogenous tablet, a tablet having two or more layers, a pouch having one or more compartments, a regular or compact powder, a granule, a paste, a gel, or a regular liquid, compact liquid, or concentrated liquid.

10. The detergent of claim 8 or 9, wherein the detergent is a laundry detergent composition, preferably a liquid or solid laundry detergent composition.

11. The detergent of any one of claims 8-10, wherein the detergent further comprises at least one other enzyme selected from the group of proteases, amylases, cellulases, lipases, xylanases, mannanases, cutinases, esterases, phosphodiesterases, phytases, DNases, pectinases, pectate lyases, pectinolytic enzymes, carbohydrases, arabinases, galactanases, xanthanases, xyloglucanases, laccases, peroxidases, and oxidases with or without a mediator.

12. The detergent of any one of the claims 8-11 further comprising at least one additive selected from the group of stabilizer, buffer, surfactant, builder, bleaching agent, mediator, anticorrosion agent, antiredeposition agent, caustic, abrasive, optical brightener, dye, pigment, and preservative.

13. A method of producing the cellulase variant of any one of the claims 1-7 comprising culturing a recombinant host cell having genetic elements configured to express a polypeptide having the amino acid sequence of the cellulase variant of any one of the claims 1-7, and recovering the polypeptide.

14. A method for antigreying, stain removal, fiber care, color care, biostoning, or biofinishing a cotton containing fabric, comprising a step of contacting the cellulase variant according to any of claims 1 to 7, or the detergent according claims 8-12, with the cotton containing fabric.

15. The method of claim 14, wherein the cotton containing fabric is a garment, fabric, or yarn containing cotton.

16. A method of generating a cellulase variant comprising the steps of:
(a) providing a template nucleic acid sequence of a template cellulase;
(b) identifying by biocomputing means active site residues and loop region residues of the template cellulase;
(c) generating variant nucleic acid sequences that encode candidate cellulase variants by modifying the template nucleic acid sequence by a modification, deletion, or addition of one or more amino acid in the active site residues and/or in loop region residues;
(d) expressing the template nucleic acid sequence in a recombinant host cell to provide template cellulase enzyme;
(e) expressing the variant nucleic acid sequences in a recombinant host cell to provide a candidate variant enzyme;
(f) testing and scoring the candidate variant enzymes, and using the template cellulase enzyme as a reference, for production level in the host cell, and antigreying performance; and
(g) selecting the candidate variant enzyme as the cellulase variant when the candidate variant enzyme reaches a predetermined scoring threshold value based on the performance in step (f).

17. The method of claim 16, wherein the template nucleic acid sequence of the template cellulase encodes SEQ ID NO: 1.
